# EUROPÄISCHE PATENTSCHRIFT

(11) **EP 1 510 199 B1**
(45) Veröffentlichungstag und Bekanntmachung des Hinweises auf die Patenterteilung: **11.10.2006**
(21) Anmeldenummer: 04019544.8
(22) Anmeldetag: 18.08.2004
(51) Int. Cl.: A61K 8/00, A61Q 5/00, A61Q 19/00

(54) **Wachsdispersionen zur Verwendung in/als Körper- und Haarpflegemittel**
Wax dispersions for use in or as haircare and bodycare preparations
Dispersions de cire et leur utilisation comme traitements capillaires et dans des produits de soin corporel

(30) Priorität: 27.08.2003 DE 10339760
(43) Veröffentlichungstag der Anmeldung: 02.03.2005
(73) Patentinhaber: Cognis IP Management GmbH, 40589 Düsseldorf (DE)
(72) Erfinder: Nieendick, Claus, 47807 Krefeld (DE); Seipel, Werner, 40723 Hilden (DE)

(56) Entgegenhaltungen:
- DE-A- 10 300 506
- DE-A- 19 837 191
- US-B1- 6 562 876
- US-B2- 6 432 419

## Beschreibung

### Gebiet der Erfindung

Die Erfindung betrifft spezifische Mischungen bestimmter Ölkörper und wasserhaltiger Wachsdispersionen, die in kosmetische und pharmazeutische Zubereitungen einarbeitbar sind, eine gute dermatologische Verträglichkeit aufweisen und insbesondere für kosmetische Formulierungen zur Haarbehandlung verwendet werden.

### Stand der Technik

Im Bereich der Körperreinigung und Körperpflege werden tensidhaltige Formulierungen eingesetzt. Der Einsatz von Tensiden ist mit dem Nachteil verbunden, dass zusammen mit der Reinigung auch eine Entfettung stattfindet, die beispielsweise zu Hautrauhigkeit und trockenem Haar führt. Zu diesem Zweck werden entsprechenden Präparaten sogenannte Rückfetter zugesetzt, bei denen es sich im einfachsten Fall um Fette, Öle oder Wachse handelt, die den Lipidgehalt von Haut und Haar wieder ausgleichen.

Die Einarbeitung solcher Stoffe in wässrige Systeme ist wegen ihrer hohen Hydrophobie oft schwierig. Eine Möglichkeit besteht darin, die Stoffe aufzuschmelzen und dann feinstverteilt einzuarbeiten, was aber auch schon bei geringen Einsatzmengen zu einem sehr aufwendigen Verarbeitungsprozess führt. In der Formulierungstechnik geht man daher in der Regel einen anderen Weg und solubilisiert die rückfettenden Wirkstoffe durch Einsatz von Lösungsvermittlern oder Hydrotropen. Hierbei handelt es sich jedoch üblicherweise um ethylenoxidhaltige Produkte, die eine geringe Neigung zum biologischen Abbau aufweisen, Viskosität und Schaumbildung nachteilig beeinflussen und außerdem zu einem trockenen Hautgefühl beitragen, da sie das hauteigene Sebum im Waschprozess mitlösen und so der Rückfettung gerade entgegen wirken.

Deswegen wird seit geraumer Zeit nach Ersatzstoffen für Silikonöle gesucht, insbesondere nach Emollient-Mischungen, die es erlauben Kosmetika ohne Silikonöle zu formulieren, ohne dabei auf das spezifische sensorische Profil dieser Stoffe zu verzichten. Stoffe, die sich als Silikonölersatz oder teilweiser Silikonölersatz eignen, um einen "build-up"-Effekt auf Haut und Haaren zu vermeiden, sind beispielsweise aus der **WO 97/47281** bekannt. Hierfür wurde der Einsatz von Ölkörpem vorgeschlagen, die ausgewählt sind aus der Gruppe der Dialkylether, der Dialkylcyclohexane, der Guerbetalkohole, der Guerbetcarbonate, der Esteröle, der Polyolpolyhydroxystearate und/oder der Hydroxycarbonsäureester. Aus der **WO 97/47282** sind kosmetische und/oder pharmazeutische Zubereitungen mit speziellen Dialkylcarbonaten und Emulgatoren bekannt, die sich durch besondere sensorische Eigenschaften auszeichnen, wobei sich die Dialkylcarbonate als gleichwertige Ersatzstoffe für Siliconöle erwiesen.

Aus der **WO 02/34216** sind Rückfettungskonzentrate bekannt, die spezifische Fettalkohole und bestimmte Wirkstoffe u.a. ausgewählt aus der Gruppe der Fettsäuren, Ölkörper, Fette, Wachse, Siliconverbindungen und Partialglyceride enthalten. Diese sind jedoch bezüglich des taktilen Gefühls noch verbesserungswürdig.

Die Aufgabe der vorliegenden Erfindung hat somit darin bestanden, ein alternatives Compound zur Herstellung von kosmetischen oder pharmazeutischen bereitzustellen, welches über rückfettende Eigenschaften für Haut und Haar verfügt. Die mit Hilfe dieses Compounds hergestellten kosmetischen oder pharmazeutischen Zubereitungen sollen weiterhin durch ein gutes Haut- und Haargefühl gekennzeichnet sein. Weiterhin sollen die Compounds kalt in die kosmetischen und pharmazeutischen Formulierungen einarbeitbar sein.

### Beschreibung der Erfindung

Gegenstand der vorliegenden Erfindung sind Wachsdispersionen mit einer durchschnittlichen Partikelgröße von 30 µm bis 100 nm umfassend (a) 15 bis 30 Gew.% einer Wachsphase ausgewählt aus Dialkyl(en)ethern, Dialkyl(en)alkoxyethern, Dialkyl(en)carbonaten, Fettalkoholen, Ethylenglykoldistearat, Stearinsäuremonoalkylester, Dicarbonsäuredistearylester, Tricarbonsäuretristearylester, Stearinsäurealkoxyalkylester oder einem beliebigen Gemisch dieser Substanzen, (b) 1 bis 10 Gew.% wenigstens eines amphoteren und/oder zwitterionischen Tensids, (c) 7 bis 15 Gew.% eines Öles ausgewählt aus Di-n-Octylether, Di-n-Octylcarbonat und/oder eines Guerbetalkohols mit 12 Kohlenstoffatomen und (d) Wasser, wobei der nicht-wäßrige Anteil der Dispersion maximal 50 Gew.% ausmacht.

### Wachsphase

Als Wachsphase eignen sich Dialkyl(en)ether, Dialkyl(en)alkoxyether, Dialkyl(en)carbonate, Fettalkohole, Ethylenglykoldistearat, Stearinsäuremonoalkylester, Stearinsäurealkoxyalkylester, Dicarbonsäuredistearylester, Tricarbonsäuretristearylester oder ein beliebiges Gemisch dieser Substanzen. Bevorzugt sind Verbindungen mit einen Stearylrest, da diese Verbindungen einen Schmelzpunkt von 50 bis 75 °C aufweisen. Besonders bevorzugt sind dabei Wachse mit einem Schmelzpunkt von 55 bis 65 °C. In einer bevorzugten Ausführungsform ist die Wachsphase ausgewählt aus der Gruppe von Verbindungen, die gebildet wird von Distearylether, PEG-4 Distearylether, Distearylcarbonat, Stearylstearat, PEG-3 Stearinsäurestearylester, Adipinsäuredistearylether, Azelainsäuredistearylether, Tristearylcitrat oder beliebigen Mischungen dieser Verbindungen. In einer ganz besonders bevorzugten Ausführungsform wird ein Ethylenglykoldistearat als Wachsphase verwendet, welches dadurch gekennzeichnet ist, daß der Fettsäureanteil des Ethylenglykoldistearats zu 70 bis 85 % aus Oktadekansäure besteht. In einer ebenfalls besonders bevorzugten Ausführungsform wird als Wachsphase ein Stearinsäurealkoxyalkylester verwendet, wobei die Alkoholkomponente des Stearinsäurealkoxyalkylesters zu mindestens 90 Gew.% aus Stearylalkohol besteht und mit 1 bis 3 Mol Ethylenoxid verethert wurde.

Üblicherweise enthalten die erfindungsgemäßen Wachsdispersionen 15 bis 30 Gew.% der Wachsphase, vorzugsweise 10 bis 25 Gew.% und in einer besonders bevorzugten Ausführungsform 20 bis 25 Gew.% einer Wachsphase.

### Amphotere und/oder zwitterionische Tenside

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bevorzugt im Sinne der vorliegenden Erfindung werden Alkylamidobetaine und insbesondere Cocamidopropylbetain. Erfindungsgemäße Wachsdispersionen enthalten 1 bis 10 Gew.% amphotere bzw. zwitterionische Tenside, insbesondere 2 bis 7 Gew.% und besonders bevorzugt 3 bis 5 Gew.%.

### Öle

Erfindungsgemäß werden als Komponente (c) Di-n-Octylether, Di-n-octylcarbonat und/oder Guerbetalkohole mit 12 Kohlenstoffatomen (bevorzugt Ethyldecanol) in Mengen von 7 bis 15 Gew.%, bevorzugt von 10 bis 13 Gew.% eingesetzt.

### Polymere

In einer weiteren bevorzugten Ausführungsform enthalten die erfindungsgemäßen Wachsdispersionen wenigstens ein Polymer in einer Menge von 0,01 bis 5Gew.% bezogen auf die Wachsdispersion. Das Polymer ist dabei vorzugsweise ausgewählt aus der Gruppe der Polyacrylate, der Polysaccharide, der Polyacrylamide oder einem beliebigen Gemisch dieser Polymere. Besonders bevorzugt sind dabei kationisch modifizierte Polysaccharide.

Der nicht-wässrige Anteil der erfindungsgemäßen Wachsdispersionen macht maximal 50 Gew.%, vorzugsweise 35 bis 45 Gew.% aus.

### Kosmetische/pharmazeutische Zubereitungen

Die erfindungsgemäßen Wachsdispersionen lassen sich kalt in kosmetische und/oder pharmazeutische Zubereitungen, insbesondere in Körper- und Haarpflegemittel einarbeiten. Sie sollen diesen Zubereitungen rückfettende Eigenschaften verleihen, so dass diese ein gutes Haut- und Haargefühl vermitteln. Hierbei handelt es sich z.B. um Körperpflege-Produkte, die als Creme, Milch, Lotion oder sprühbare Emulsion formuliert werden, um Produkte zur Eliminierung des Körpergeruchs oder zum Schutz gegen UV-Strahlung. Die erfindungsgemäße Zusammensetzung lässt sich auch in tensidhaltigen Formulierungen wie z. B. Schaum- und Duschbädem, Haarshampoos, Haarkuren und Pflegespülungen einsetzen. Vorzugsweise sind derartige Zubereitungen frei von Silikonen. Ein weiterer Gegenstand der vorliegenden Patentanmeldung ist somit auch die Verwendung der Wachsdispersionen in Körper- und/oder Haarpflegemitteln.

Die kosmetischen Mittel können in Form von Emulsionen oder Dispersionen vorliegen, die Wasser und Ölphase nebeneinander enthalten. Bevorzugte kosmetische Zusammensetzungen sind solche in Form einer W/O- oder O/W-Emulsion mit den üblichen, dem Fachmann geläufigen, Konzentrationen an Ölen/Fetten/Wachsen, Emulgatoren, Wasser und den in der Kosmetik üblichen, weiteren Hilfs- und Zusatzstoffen.

Je nach Applikationszweck enthalten die kosmetischen Formulierungen eine Reihe weiterer Hilfs- und Zusatzstoffe wie beispielsweise oberflächenaktive Substanzen (Tenside, Emulgatoren), weitere Ölkörper, Perlglanzwachse, Konsistenzgeber, Verdickungsmittel, Überfettungsmittel, Stabilisatoren, Polymere, Siliconverbindungen , Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungs-mittel), Hydrotrope, Solubilisatoren, Konservierungsmittel, Parfümöle, Farbstoffe etc., die nachstehend exemplarisch aufgelistet sind.

Die Mengen der jeweiligen Zusätze richten sich nach der beabsichtigten Verwendung.

### Oberflächenaktive Substanzen

Als oberflächenaktive Stoffe können anionische, nichtionische, kationische und/oder amphotere bzw. zwitterionische Tenside bzw. Emulgatoren oder ein beliebiges Gemisch dieser Tenside/Emulgatoren enthalten sein. Der Gehalt oberflächenaktiver Substanzen hängt von der Art der Formulierung ab, übersteigt aber üblicherweise 20 Gew.-% nicht. In tensidhaltigen kosmetischen Zubereitungen, wie beispielsweise Duschgelen, Schaumbädern, Shampoos etc. ist vorzugsweise wenigstens ein anionisches Tensid enthalten, in Cremes und Lotionen für die Körperpflege sind vorzugsweise nichtionische Tenside/Emulgatoren enthalten.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Polyglycerinester, Alkylphenolpolyglycolether, Fettsäurepolyglycolester, Fettsäureamidpoly-glycolether, Fettaminpolyglycolether, alkoxylierte Triglyceride, Mischether bzw. Mischformale, Alk(en)yloligoglykoside bzw. Glucoronsäurederivate - geg ebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper sind üblicherweise in einer Gesamtmenge von 1 - 50 Gew.-%, vorzugsweise 5 - 25 Gew.-% und inbesondere 5 - 15 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆₋C₂₂-Fettsäuren mit linearen oder verzweigten C₆-C₂₂-Fettalkoholen bzw. Ester von verzweigten C₆-C₁₃₋Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Eru-cyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀Fettsäuren, flüssige Mono-/Di-/Triglyceridmischungen auf Basis von C₆-C₁₈₋Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE) oder Ringöffnungsprodukte von epoxidierten Fettsäureestem mit Polyolen.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im Wesentlichen aus gemischten Glycerinestem höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Verdickungsmittel

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyacrylate, (z. B. Carbopole® und Pemulen-Typen von Goodrich; Synthalene® von Sigma; Keltrol-Typen von Kelco; Sepigel-Typen von Seppic; Salcare-Typen von Allied Colloids und Cosmedia® SP und SPL von Cognis), Polyacrylamide, Polymere, Polyvinylalkohol, Polyvinylpyrrolidon und Bentonite wie z. B. Bentone® Gel VS-5PC (Rheox). Weiter in Frage kommen Elektrolyte wie Kochsalz und Ammoniumchlorid.

### Stabilisatoren

Als Stabilisatoren können Metallsalze von Fettsäuren, wie z. B. Magnesium-, Aluminium- und/oder Zinkstearat bzw. -ricinoleat eingesetzt werden.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol® 1789) und 2-Cyano-3,3-phenylzimtsäure-2-ethyl-hexylester (Octocrylene) sowie Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtem wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide bzw. Salze in Frage. Beispiele für geeignete Metalloxide sind insbesondere Zinkoxid und Titandioxid. Als Salze können Silicate (Talk), Bariumsulfat oder Zinkstearat eingesetzt werden. Die Oxide und Salze werden in Form der Pigmente für hautpflegende und hautschützende Emulsionen verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Hydrotrope

Zur Verbesserung des Fließverhaltens können ferner Hydrotrope, wie beispielsweise Ethanol, Iso-propylalkohol, oder Polyole eingesetzt werden. Polyole, die hier in Betracht kommen, besitzen vorzugsweise 2 bis 15 Kohlenstoffatome und mindestens zwei Hydroxylgruppen.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

### Farbstoffe

Als Farbstoffe können die für kosmetische Zwecke geeigneten und zugelassenen Substanzen verwendet werden. Beispiele sind Kochenillerot A (C.l. 16255), Patentblau V (C.l.42051), Indigotin (C.l.73015), Chlorophyllin (C.l.75810), Chinolingelb (C.l.47005), Titandioxid (C.l.77891), Indanthrenblau RS (C.l. 69800) und Krapplack (C.l.58000). Diese Farbstoffe werden üblicherweise in Konzentrationen von 0,001 bis 0,1 Gew.-%, bezogen auf die gesamte Mischung, eingesetzt.

### Beispiele

Die folgenden kosmetischen Zubereitungen wurden unter Verwendung der erfindungsgemäßen Wachsdispersionen hergestellt und zeichnen sich durch ein hervorragenden Haut- und Haargefühl, sowie gute rückfettende Eigenschaften aus. Alle Angaben verstehen sich in Gew.%.

| **Inhaltsstoffe** | **1** | **2** | **3** | **4** | **5** | **6** |
|---|---|---|---|---|---|---|
| ***lNCI*** | **Placebo** | | | | | |
| **Texapon N 70** | 13,0 | 13,0 | 13,0 | 13,0 | 13,0 | 13,0 |
| *Sodium laureth sulfate* | | | | | | |
| **Dehyton PK 45** | 4,5 | 7,5 | 7,5 | 7,5 | 7,5 | 7,5 |
| *Cocamidopropyl betaine* | | | | | | |
| **Carbopol Aqua SF-1** | 3,0 | 3,0 | 3,0 | 3,0 | 3,0 | 2,9 |
| *Acrylat polymer* | | | | | | |
| **Polymer JR 400** | - | 0,2 | 0,2 | 0,2 | 0,2 | 0,2 |
| *Polyquaternium-10* | | | | | | |
| **Lamesoft KE3999** | 5,0 | - | - | - | - | - |
| *20 Gew. % Ethylene glycol distearate (and)* | | | | | | |
| *10 Gew. % Cocamidopropyl betaine (and)* | | | | | | |
| *5 Gew.* % *Dioctyl ether* | | | | | | |
| **Lamesoft KE4003** | - | - | - | - | - | 5,0 |
| *20 Gew. % Ethylene glycol distearate (and)* | | | | | | |
| *10 Gew. % Cocamidopropyl betaine (and)* | | | | | | |
| *8 Gew.* % *Dioctyl ether* | | | | | | |
| 1*% Carbopol Aqua SF1* | | | | | | |
| **Lamesoft KE3809** | - | 5,0 | 5,0 | - | - | - |
| *20 Gew. % Distearyl carbonate (and)* | | | | | | |
| *10 Gew. % Cocamidopropyl betaine (and)* | | | | | | |
| *3 Gew. % Sodium Laureth Sulfate (and)* | | | | | | |
| *8 Gew. % Dioctyl carbonate (and)* 2 *Gew.* % | | | | | | |
| *Octyldodecanol* | | | | | | |
| **Lamesoft KE3890** | - | - | - | 5,0 | 5,0 | - |
| *10 Gew. % Distearyl carbonate (and)* | | | | | | |
| 10 *Gew. % Stearyl stearate (and)* | | | | | | |
| 10 *Gew. %* Coco *betaine (and)* | | | | | | |
| *5 Gew% Cocoglucosid (and)* | | | | | | |
| *8 Gew. % Dioctylether* | | | | | | |
| **Parfum** | 0,3 | 0,3 | 0,3 | 0,3 | 0,3 | 0,3 |
| **Konservierungsmittel** | 0,1 | 0,1 | 0,1 | 0,1 | 0,1 | 0,1 |
| **Kochsalz** | 1,6 | 1,5 | 1,3 | 1,3 | 0,1 | 0,1 |
| **Wasser** | ad 100 | | | | | |
| **pH-Wert** | 6,49 | 6,5 | 6,52 | 6,52 | 6,5 | 6,45 |

Für die o.g. Rezepturen 1 bis 8 wurde jeweils die Trocken- und Naßkämmbarkeit bestimmt.

Zur Ermittelung der Kämmarbeit wurden gebleichte Haarsträhnen mit dem zu untersuchenden Produkt (1g Produkt/1g Haar) behandelt, Einwirkzeit 5 min, dann ausgespült (Leitungswasser 38°C, 1 l/min). Anschließend wurden in einer speziellen Messapparatur die Kräfte bestimmt, die beim Kämmen der Haare auftreten. Aus den gemessenen Kraft-Weg-Verläufen wurde die Kämmarbeit durch Integration ermittelt. Der Quotient aus Kämmarbeit für unbehandelte und der Kämmarbeit an behandelten Haarsträhnen ergibt die sogenannte residuale Kämmarbeit. Jede Messung wurde an 20 Haarsträhnen durchgeführt.

Zur Bestimmung der Trockenkämmarbeit wurden 18 cm lange Haarsträhnen (2 g) verwendet, die vor der Messung in der abgeschlossenen Apparatur für 12 h klimatisiert wurden. Die Kämmung erfolgte mit Kämmen, die einen Zinkenabstand von 2,6 mm aufweisen. Für die Nasskämmarbeit wurden 15 cm Strähnen (1g) eingesetzt, die Kämmmessung erfolgte unmittelbar nach dem Ausspülen. Hier wurden Kämme mit einem Zinkenabstand von 0,7 mm eingesetzt. Um konstante Bedingungen zu gewährleisten, befand sich die gesamte Apparatur in einer Einhausung, in der eine Temperatur von 30°C und eine relative Luftfeuchte von 40% eingestellt wurde.

| **Rezeptur** | Naß kämmarbeit **vorher [mJ]** | Naß kämmarbeit **nachher [mJ]** | Restkämmarbeit [%] |
|---|---|---|---|
| **1 Placebo** | 108.51 | 100.54 | 92.66 |
| **2** | 100.32 | 61.11 | 60.09 |
| **3** | 106.55 | 63.77 | 59.85 |
| **4** | 108.25 | 62,40 | 57.64 |
| **5** | 102.60 | 60.10 | 58.57 |
| **6** | 105,33 | 66.63 | 63.26 |

Die Messreihen sind zu 95% signifikant unterschieden in Bezug auf die Nasskämmarbeit vorher.

| **Rezeptur** | Trockenkämmarbeit **vorher [mJ]** | Trockenkämmarbeit **nachher [mJ]** | Restkämmarbeit [%] |
|---|---|---|---|
| **1 Placebo** | 79.51 | 67.9 | 85.39 |
| **2** | 76.91 | 50.30 | 63.10 |
| **3** | 74.60 | 48.75 | 65.34 |
| **4** | 78,25 | 49.05 | 62.68 |
| **5** | 72.94 | 45.15 | 61.90 |
| **6** | 77.88 | 50.40 | 64.71 |

Die Messreihen sind zu 95% signifikant unterschieden in Bezug auf die Trockenkämmarbeit vorher.

Den Ergebnissen der Naß- und Trockenkämmbarkeit ist zu entnehmen, daß die erfindungsgemäßen Kombinationen die stärkste Erniedrigung der Kämmarbeit bewirken.

## Patentansprüche

1. Wachsdispersion mit einer durchschnittlichen Partikelgröße von 30 µm bis 100 nm umfassend
(a) 15 bis 30 Gew.% einer Wachsphase ausgewählt aus Dialkyl(en)ethern, Dialkyl(en)alkoxyethern, Dialkyl(en)carbonaten, Fettalkoholen, Ethylenglykol-distearat, Stearinsäuremonoalkylester, Stearinsäurealkoxyalkylester, Dicarbonsäuredistearylester, Tricarbonsäuretristearylester oder einem beliebigen Gemisch dieser Substanzen,
(b) 1 bis 10 Gew.% wenigstens eines amphoteren und/oder zwitterionischen Tensids,
(c) 7 bis 15 Gew.% eines Öles ausgewählt aus Di-n-Octylether, Di-n-Octylcarbonat und/oder eines Guerbetalkohols mit 12 Kohlenstoffatomen und
(d) Wasser,
wobei der nicht-wässrige Anteil der Dispersion maximal 50 Gew.% ausmacht.

2. Wachsdispersion gemäß Anspruch 1, **dadurch gekennzeichnet, dass** die Wachsphase ausgewählt ist aus der Gruppe, die gebildet wird von Distearylether, PEG-4 Distearylether, Distearylcarbonat, Stearylstearat, Adipinsäuredistearylester, Azelainsäuredistearylester, PEG-3 Stearinsäurestearylester und Tristearylcitrat.

3. Wachsdispersion gemäß Anspruch 1, **dadurch gekennzeichnet, dass** der Fettsäureanteil des Ethylenglykoldistearats zu 70 bis 85 % aus Oktadekansäure besteht.

4. Wachsdispersion gemäß Anspruch 1, **dadurch gekennzeichnet, daß** die Alkoholkomponente des Stearinsäurealkoxyalkylesters zu mindestens 90 Gew.% aus Stearylalkohol besteht und mit 1 bis 3 Mol Ethylenoxid verethert wurde.

5. Wachsdispersion gemäß wenigstens einem der Ansprüche 1 bis 4, **dadurch gekennzeichnet, dass** sie wenigstens ein Polymer, vorzugsweise in einer Menge von 0,01 bis 5 Gew.% bezogen auf die Wachsdispersion enthält.

6. Wachsdispersion gemäß wenigstens einem der Ansprüche 1 bis 5, **dadurch gekennzeichnet, dass** das Polymer ausgewählt ist aus der Gruppe der Polyacrylate, der Polysaccharide, der Polyacrylamide oder einem beliebigen Gemisch dieser Polymere.

7. Verwendung einer Wachsdispersion gemäß einem der Ansprüche 1 bis 6 als Körperpflegemittel oder zur Herstellung von Körperpflegemitteln.

8. Verwendung einer Wachsdispersion gemäß einem der Ansprüche 1 bis 6 als Haarpflegemittel oder zur Herstellung von Haarpflegemitteln.

## Claims

1. Wax dispersions with an average particle size of 30 µm to 100 nm containing
(a) 15 to 30% by weight of a wax phase selected from dialkyl(ene)ethers, dialkyl(ene) alkoxyethers, dialkyl(ene) carbonates, fatty alcohols, ethylene glycol distearate, stearic acid monoalkyl esters, stearic acid alkoxyalkyl esters, dicarboxylic acid distearyl esters, tricarboxylic acid tristearyl esters or a mixture of these substances,
(b) 1 to 10% by weight of at least one amphoteric and/or zwitterionic surfactant,
(c) 7 to 15% by weight of an oil selected from di-n-octyl ether, di-n-octyl carbonate and/or a C₁₂ Guerbet alcohol and
(d) water,
the non-aqueous part of the dispersion making up at most 50% by weight.

2. A wax dispersion as claimed in claim 1, **characterized in that** the wax phase is selected from the group consisting of distearyl ether, PEG-4 distearyl ether, distearyl carbonate, stearyl stearate, adipic acid distearyl ester, azelaic acid distearyl ester, PEG-3 stearic acid stearyl ester and tristearyl citrate.

3. A wax dispersion as claimed in claim 1, **characterized in that** 70 to 85% of the fatty acid component of the ethylene glycol distearate consists of octadecanoic acid.

4. A wax dispersion as claimed in claim 1, **characterized in that** at least 90% by weight of the alcohol component of the stearic acid alkoxyalkyl ester consists of stearyl alcohol, the alcohol component having been etherified with 1 to 3 mol ethylene oxide.

5. A wax dispersion as claimed in at least one of claims 1 to 4, **characterized in that** it contains at least one polymer, preferably in a quantity of 0.01 to 5% by weight, based on the wax dispersion.

6. A wax dispersion as claimed in at least one of claims 1 to 5, **characterized in that** the polymer is selected from the group of polyacrylates, polysaccharides, polyacrylamides or a mixture of these polymers.

7. The use of the wax dispersion claimed in any of claims 1 to 6 as a body care preparation or for the production of body care preparations.

8. The use of the wax dispersion claimed in any of claims 1 to 6 as a hair care preparation or for the production of hair care preparations.

## Revendications

1. Dispersion de cire ayant une taille particulaire moyenne allant de 30 µm à 100 nm, comprenant :
(a) de 15 à 30 % en poids d'une phase cireuse choisie parmi les éthers de dialkyl(èn)e, les alcoxyéthers de dialkyl(èn)e, les carbonates de dialkyl(èn)e, les alcools gras, le distéarate d'éthylène glycol, les monoalkylesters d'acide stéarique, les alcoxyalkylesters d'acide stéarique, les distéarylesters d'acides dicarboxyliques, les tristéarylesters d'acides tricarboxyliques ou un mélange quelconque de ces substances,
(b) de 1 à 10 % en poids d'au moins un tensioactif amphotère et/ou zwitterrionique,
(c) de 7 à 15 % en poids d'une huile choisie parmi l'éther di-n-octylique, le carbonate di-n-octylique et/ou un alcool de Guerbet ayant 12 atomes de carbone, et
(d) de l'eau,
la partie non aqueuse de la dispersion représentant au plus 50 % en poids.

2. Dispersion de cire selon la revendication 1,
**caractérisée en ce que**
la phase cireuse est choisie dans le groupe constitué par l'éther distéarylique, le PEG-4 éther distéarylique, le carbonate distéarylique, le stéarate stéarylique, le distéarylester d'acide adipique, le distéarylester d'acide azélaïque, le PEG-3 stéarylester d'acide stéarique et le tristéarylcitrate.

3. Dispersion de cire selon la revendication 1,
**caractérisée en ce que**
la partie d'acide gras du distéarate d'éthylène glycol se compose d'acide octadécanoïque à raison de 70 à 85 %.

4. Dispersion de cire selon la revendication 1,
**caractérisée en ce que**
le composant alcool de l'alcoxyalkylester d'acide stéarique se compose, à raison d'au moins 90 % en poids, d'alcool stéarylique et a été éthérifié avec de 1 à 3 mole d'oxyde d'éthylène.

5. Dispersion de cire selon au moins l'une des revendications 1 à 4,
**caractérisée en ce qu'**
elle contient au moins un polymère, de préférence en une quantité allant de 0,01 à 5 % en poids par rapport à la dispersion de cire.

6. Dispersion de cire selon au moins l'une des revendications 1 à 5,
**caractérisée en ce que**
le polymère est choisi dans le groupe constitué par les polyacrylates, les polysaccharides, les polyacrylamides ou un mélange quelconque de ces polymères.

7. Utilisation d'une dispersion de cire selon l'une des revendications 1 à 6, comme produit de soins corporels ou pour la préparation de produits de soins corporels.

8. Utilisation d'une dispersion de cire selon l'une des revendications 1 à 6, comme produit de soins capillaires ou pour la préparation de produits de soins capillaires.
